# EUROPEAN PATENT APPLICATION

(11) **EP 1 623 981 A1**
(43) Date of publication of application: **08.02.2006**
(21) Application number: 04730680.8
(22) Date of filing: 30.04.2004
(51) Int. Cl.: C07D 277/64, C07D 417/14, C07D 417/06, A61K 31/428, A61K 31/4439, A61P 33/00

(54) **ANTI-LEISHMANIA AGENT**

(30) Priority: 06.05.2003 JP 2003128454
(71) Applicant: Japan Science and Technology Corporation, Kawaguchi-shi, Saitama 332-0012 (JP)
(72) Inventor: IHARA, Masataka, 9820021 (JP); TAKASU, Kiyosei, Sendai-shi, Miyagi, 982-0012 (JP); TERAUCHI, Hiroki, Sendai-shi, Miyagi 982-0832 (JP); SEKITA, Setsuko, 3050035 (JP); TAKAHASHI, Marii, 2640026 (JP)
(74) Representative: Harding, Charles Thomas
(86) International application number: PCT/JP2004/006315
(87) International publication number: WO 2004/108695

(57) **Abstract**

The present invention is to provide a new anti-leishmania agent with fewer side effects, having a high cell proliferation-inhibiting effect to leishamia protozoa, and also easy to manufacture at a low cost. A compound wherein a heterocycle with a conjugated system and a nitrogen atom and a heterocycle with a nitrogen atom and a sulfur atom are bound by a carbon chain with an ethylene group, that is a compound wherein a specific 5- to 8-membered heterocyclic ring and a specific 5-to 8-membered heterocycle having a conjugated system are bound via a vinylene group, more particularly a specific rhodacyanine dye compound is used as an active ingredient of the anti-leishmania agent.

## Description

### Technical Field

The present invention relates to an anti-leishmania agent containing a specific compound useful for the prevention or treatment of Leishmaniasis as an active ingredient, and more particularly to an anti-malaria agent containing rhodacyanine dye compound as an active ingredient.

### Background Art

Leishmaniasis is a tropical parasite infection which is caused by paratisitism of protozoa of Leishamia genus to macrophages of hosts including humans, and propagates being mediated by sand flies living mainly in desert. It is nominated as one of the six major tropical diseases by WHO, and almost all the Leishaminasis-patients of the world are living in Africa, Middle East, Central and South Africa and Asia (about 12 million of patients/year) and about 350 millions persons are at risk of infection. Most of them are living in developing countries having difficulties to buy expensive and rare drugs.

Leishmaniasis is classified in three main groups: cutaneous leishmaniasis (oriental sore), mucocutaneous leishmaniasis, and visceral leishmaniasis (kala-azar). Onset of kala-azar, a visceral leishmaniasis, occurs by infection of 3 types of Leishmania donovani group. Oriental phymas of cutaneous and mucocutanesous leishmaniasis distributed on the coast of the Mediterranean Sea, Africa and Middle East, becomes a ulcer by generating phyma to skin, by an infection of three types of L. tropica group, sometime causing a remote transfer of the disease or generating a leper knot-like diffuse lesion. When it is an infection caused by four types of L. braziliensis group, a ulcer is formed to the nasal or oral mucous membrane from the first dermal lesion, further causing deformation thereof. Moreover, when it is an infection caused by three types of L. mexicana group, a legion limited to skin, a little different from that of oriental phyma is generated. These various types of leishmaniasis may be accompanied with a risk of high lethality if not treated sufficiently. Leishmania protozoa are of great variety, and from an immunological point of view, as the antigenicity differs among regions, even for similar pathologies, the development of vaccine is difficult, and a chemical treatment is in great need.

Currently, a pentavalent antimonial agent, for example pentostam to be administered intravenously or intramuscularly, is used for treating leishmaniasis as a first choice. However, its high cost as well as the side effects due to its high toxicity is a problem. Additionally, drug-resistant protozoa have emerged in Indian subcontinent and are causing further serious problems. When the effectivity of antimonial agent is doubtful, isethionate diamidine compounds such as pentamidine (4,4'-(pentamethylenedioxy) dibenzamidine) are used as an anti-infective drug effective to Pneumocystis carinii (see for example, Published Japanese translation of PCT international publication No. 9-501653). Moreover, macrolide antibiotics such as antifungal antibiotic Amphotericin B are used secondarily as intravenous injection to antifungal infections or mucocutaneous leishmaniasis. However, the effectivity of these agents is inferior to that of the pentavalent antimonial agent. Moreover, these agents are expensive and various side effects have been reported.

Moreover, as for treating agents of leishmniasis, antiprotozoal agents containing germacrane and guainane-type sesquiterpenoid compounds being extracted, purified and separated from Elephantopus mollis H. B. K. (see for example, Japanese Laid-Open Patent Application No. 2001-226369); or leishmaniasis-treating agents containing glucopyranose derivative as active ingredient (see for example, Japanese Laid-Open Patent Application No. 11-106394) are known.

However, these treating agents have problems such as being difficult to prepare, or to manufacture massively, thus being hardly versatile.

The object of the present invention is to provide a new anti-leishmania agent with fewer side effects, having high cell proliferation-inhibiting effect against Leishmaniasis protozoa, and being easy to manufacture.

The present inventors have found that a compound wherein a heterocycle with a conjugated system and a nitrogen atom and a heterocycle with a nitrogen atom and a sulfur atom are bound by a carbon chain with an ethylene group, is useful as an anti-leishmania agent with fewer side effects, having excellent efficacy to leishmaniasis caused by various leishmaniasis protozoa. Further, the present inventors synthesized various rhodacyanine dye compounds having different substituted groups at a specific rhodanine core (4-oxothiazolidine ring) and analyzed the anti-leishmania activity, and they have found that the rhodacyanine dye compounds wherein a heterocycle with nitrogen is bound to a specific position of the 4-oxothiazolidine ring has an extremely high anti-leishmania activity, with fewer side effects. The present invention has been thus completed.

### Disclosure of the Invention

In other words, the present invention is related to: a compound shown by formula (I) (a)

### (formula 9)

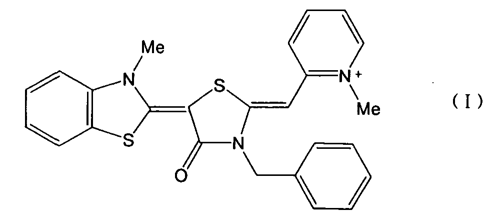

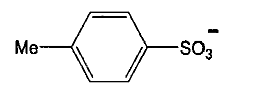

("1"); a compound shown by formula (I) (b)

### (formula 10)

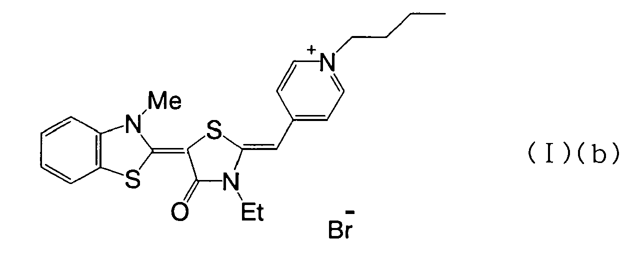

("2"); a compound shown by formula (I) (c)

### (formula 11)

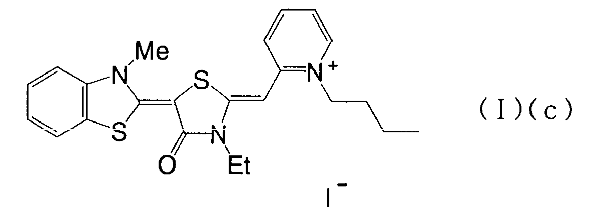

("3"); a compound shown by formula (I) (d) 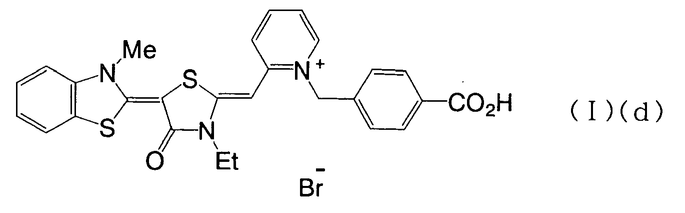 ("4"); an anti-leishmania agent containing a compound shown by general formula (II) as an active ingredient 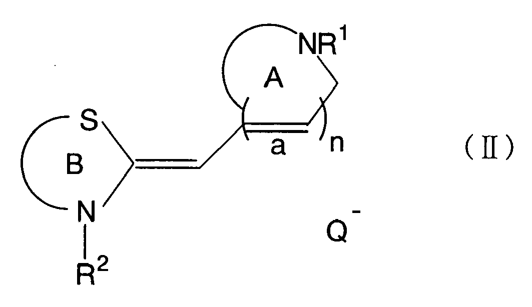 [wherein R¹ represents an alkyl group with 1 to 8 carbon atoms having unsubstituted or substituted group, an aryl group with 6 to 8 carbon atoms having unsubstituted or substituted group, or a heterocyclic group having unsubstituted or substituted group; R² represents a hydrogen atom, an alkyl group with 1 to 8 carbon atoms having unsubstituted or substituted group, an aryl group with 6 to 8 carbon atoms having unsubstituted or substituted group; or a heterocyclic group having unsubstituted or substituted group; n represents an integer of 0, 1 or 2; A and B represent independently an atom group necessary to form a 5- to 8-membered heterocycle having unsubstituted or substituted group; a represents a conjugated system; and Q represents a pharmaceutically acceptable anion] ("5"); the anti-leishmania agent according to "5", wherein the compound shown by general formula (II) is a rhodacyanine dye compound shown by general formula (III)

### (formula 14)

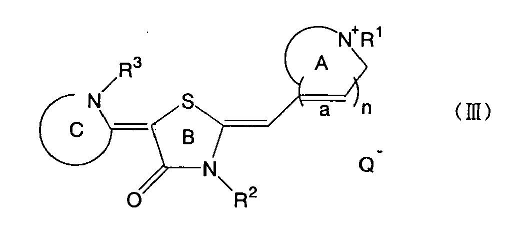

[wherein R¹ represents an alkyl group with 1 to 8 carbon atoms having unsubstituted or substituted group, an aryl group with 6 to 8 carbon atoms having unsubstituted or substituted group, or a heterocyclic group having unsubstituted or substituted group; R² and R³ represent independently a hydrogen atom, an alkyl group with 1 to 8 carbon atoms having unsubstituted or substituted group, an aryl group with 6 to 8 carbon atoms having unsubstituted or substituted group; or a heterocyclic group having unsubstituted or substituted group; n represents an integer of 0, 1 or 2; A and C represent independently an atom group necessary to form a 5- to 8-membered heterocycle having unsubstituted or substituted group; a represents a conjugated system; and Q represents a pharmaceutically acceptable anion] ("6"); the anti-leishmania agent according to "6", wherein the rhodacyanine dye compound shown by general formula (III) is a rhodacyanine dye compound shown by any one of formulae (I) (a) to (d), or formulae (IV) (a) to (m)

### (formula 15)

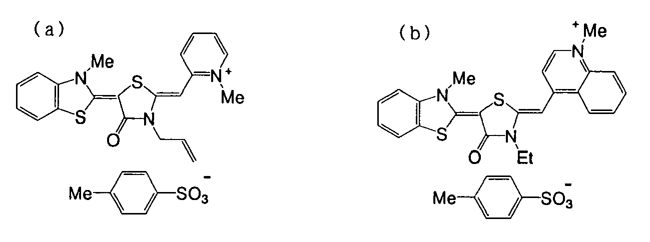

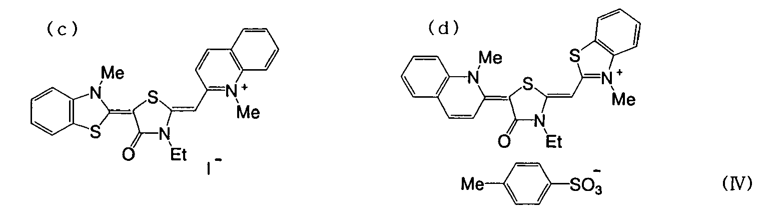

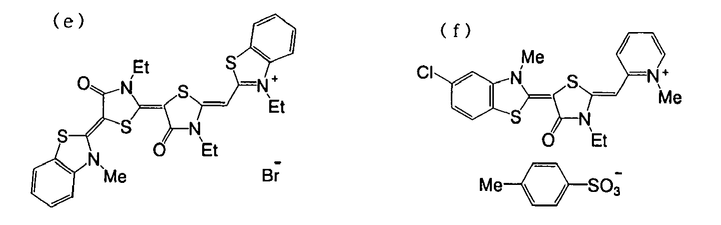

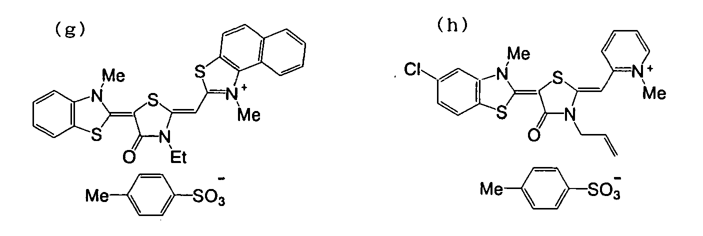

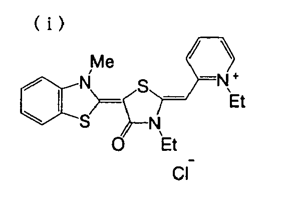

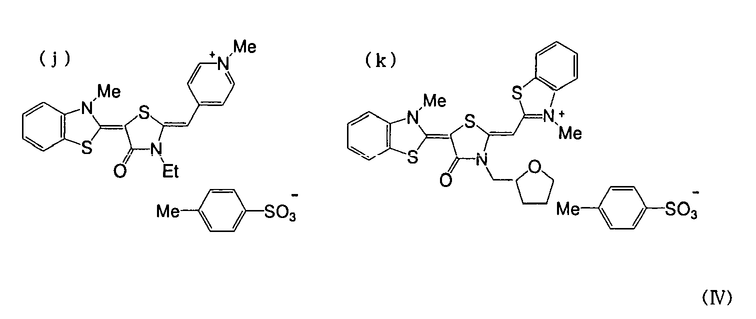

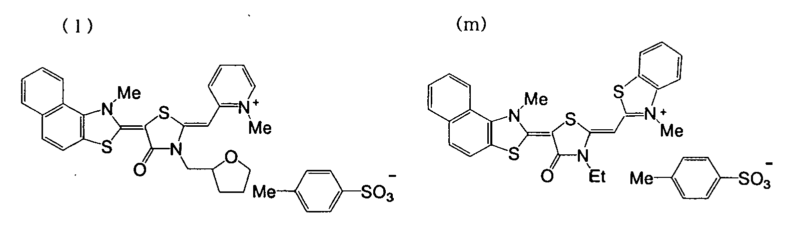

("7"); or the anti-leishmania agent according to "5", wherein the compound shown by general formula (II) is a compound shown by formula (V) (a) or (b) 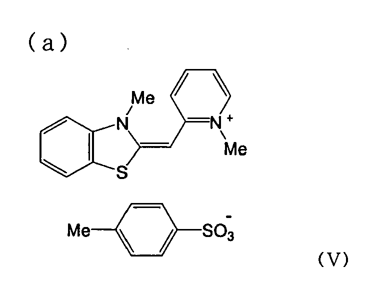 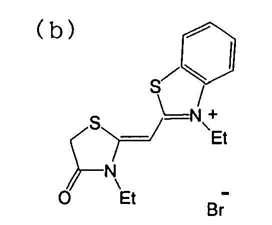 ("8").

### Brief Description of Drawings

Fig. 1 is a picture showing the anti-leishmania activity at different concentration levels of the active ingredient of the anti-leishmania agent of the present invention.
Fig. 2 is a picture showing the anti-leishmania activity at different concentration levels of the active ingredient of the anti-leishmania agent of the present invention.
Fig. 3 is a picture showing the anti-leishmania activity at different concentration levels of the active ingredient of the anti-leishmania agent of the present invention.
Fig. 4 is a picture showing the anti-leishmania activity at different concentration levels of the active ingredient of the anti-leishmania agent of the present invention.

### Best Mode of Carrying Out the Invention

The anti-leishmania agent of the present invention is not particularly limited as long as it contains a compound shown by general formula (II) as an active ingredient [wherein R¹ represents an alkyl group with 1 to 8 carbon atoms having unsubstituted or substituted group, an aryl group with 6 to 8 carbon atoms having unsubstituted or substituted group, or a heterocyclic group having unsubstituted or substituted group; R² represents a hydrogen atom, an alkyl group with 1 to 8 carbon atoms having unsubstituted or substituted group, an aryl group with 6 to 8 carbon atoms having unsubstituted or substituted group; or a heterocyclic group having unsubstituted or substituted group; n represents an integer of 0, 1 or 2; A and B represent independently an atom group necessary to form a 5- to 8-membered heterocycle having unsubstituted or substituted group; a represents a conjugated system; and Q represents a pharmaceutically acceptable anion]. In the compound shown by general formula (II), as for the alkyl group with 1 to 8 carbon atoms represented by R¹ or R², it can be one having not only a straight chain but also a branched-chain, and can be exemplified by methyl group, ethyl group, n-propyl group, isopropyl group, n-butyl group, s-butyl group, t-butyl group, isobutyl group, n-pentyl group, s-pentyl group, isopentyl group, neopentyl group, n-hexyl group, 2-methyl-n-pentyl group, n-heptyl group, 2-methyl-n-hexyl group, and n-octyl group. As for aryl group with 6 to 8 carbon atoms, examples include phenyl group, o-tryl group, m-tryl group, p-tryl group, 2,4-xyl group, 3,5-xyl group,benzyl group, phenetyl group, and α-methylbenzyl group can be exemplified. As for the substituted group to be bound to the alkyl group with 1 to 8 carbon atoms or the aryl group with 6 to 8 carbon atoms represented by R¹ or R², halogen atom, hydroxy group, oxo group, alkyl group, alkenyl group, alkynyl group, alkoxy group, alkoxycarbonyl group, carboxyl group, alkylcarbonyl group, arylcarbonyl group, aryl group, aralkyl group and amino group can be exemplified.

Further, as for the heterocyclic group represented by R¹ or R², a 5-membered heterocyclic group such as pyrrolidine, pyrrole, pyrazole, imidazole, thiazole, isothiazole, 2-thiazoline, thiazolidine, furan, tetrahydrofuran, 1,3-dioxolane, thiophene, tetrahydrothiophene; or a 6-membered heterocyclic group such as pyridine, piperidine, pyridazine, pyrimidine, pyradine, piperadine, 2H-1,4-thiadine, 4H-1,4-thiadine, 4H-1,4-oxadine, 2H-pyran, 4H-pyran, tetrahydropyran, 1,4-dioxan can be specifically exemplified. Further, as for the substituted group to be bound to the heterocycle represented by R¹ or R², besides the same substituted groups for alkyl group with 1 to 8 carbon atoms or aryl group with 6 to 8 carbon atoms, it can be a group forming ortho-condensed ring by condensing to a heterocycle; and as for an ortho-condensed ring formed with a heterocycle, indazole, benzoimidazole, benzothiazole, benzoxazole, 1,2-benzoisoxazole, quinoline, isoquinoline, cinnoline, quinazoline, quinoxaline, phthalazine, 1,8-naphthyridine can be specifically exemplified.

As for the heterocycle with a conjugated system a shown by any integer n of 0, 1 or 2, formed with A representing the atom group necessary to form 5- to 8-membered heterocycle in the above general formula (II), specifically, pyrrole, pyrazole, imidazole, 2-pyrazoline, 1H-1,2,3-triazole, 1H-1,2,4-triazole, 2H-1,2,3-triazole, 4H-1,2,4-triazole, 1H-tetrazole, thiazole, isothiazole, 2-thiazoline, oxazole, isoxazole, 1,2,5-oxadiazole, pyridine, pyridazine, pyrimidine, pyradine, 2H-1,4-thiadine, 4H-1,4-thiadine, 4H-1,4-oxadine, azepin, 1,n-diazepin (n=2-4) and azocine can be exemplified. Moreover, as for the substituted group to be bound to the heterocycle formed with the atom group shown by A, the same substituted groups to be bound to the above R¹ or R² can be exemplified, and it can be a group condensing to a heterocycle to form an ortho-condensed ring; and as for an ortho-condensed ring formed with a heterocycle, indole, indazole, benzoimidazole, benzothiazole, benzoxazole, 1,2-benzoisoxazole, quinoline, isoquinoline, cinnoline, quinazoline, quinoxaline, phtalazine, and 1,8-naphthyridine can be specifically exemplified.

As for the heterocycle formed with B representing the atom group necessary to form a 5-to 8-membered heterocycle in the above general formula (II), there are no specific limitation as long as sulfur atom and nitrogen atom are placed at the 1, 3 position, and specific examples include a 6-membered heterocycle such as thiazolidine, perhydro-1,3-thiadine, tetrahydro-1,3-thiadine; a 7-membered heterocycle such as perhydro-1,3-thiazepin, dihydro-1,3-thiazepin, tetrahydro-1,3-thiazepin; or a 8-membered heterocycle such as perhydro-1,3-thiazocine, dihydro-1,3-thiazocine, tetrahydro-1,3-thiazocine. Further, as for the substituted group to be bound to the heterocycle formed with the atom group represented by B, the same groups as the above substituted groups to be bound to R¹ and R² can be exemplified, and it can be a group condensing to a heterocycle to form an ortho-condensed ring; as for the ortho-condensing ring formed with the heterocycle, specifically, benzothiazoline, perhydro-1,3-benzo[e]thiadine, perhydro-1,3-benzo[f]thiazepin, perhydro-1,3-benzo[g]thiazocine can be exemplified.

As for the heterocycle formed with B representing the atom group necessary to form a 5- to 8-membered heterocycle in the above general formula (II), thiazolidine is preferable and 4-oxothiazolidine ring having an oxo group at 4 position is more preferable. As for the compound having 4-oxothiazolidine ring, it is preferable to be a rhodacyanine dye compound shown by general formula (III). In general formula (III), R¹ represents an alkyl group with 1 to 8 carbon atoms having unsubstituted or substituted group, an aryl group with 6 to 8 carbon atoms having unsubstituted or substituted group, or a heterocyclic group having unsubstituted or substituted group; R² and R³ represent independently a hydrogen atom, an alkyl group with 1 to 8 carbon atoms having unsubstituted or substituted group, an aryl group with 6 to 8 carbon atoms having unsubstituted or substituted group, or a heterocyclic group having unsubstituted or substituted group; as for the alkyl group with 1 to 8 carbon atoms, the aryl group with 6 to 8 carbon atoms, the heterocyclic group represented by R¹, R² and R³, and the substituted group of the alkyl group, aryl group and heterocyclic group, the same group as those represented by R¹ and R² in general formula (II) can be exemplified. Moreover, as for the heterocycle bound by double bound to the 5 position of 4-oxothiazolidine ring in general formula (III), and formed with C representing the atom group necessary to form a 5- to 8-membered heterocycle having unsubstituted or substituted group, pyrrole, pyrazole, imidazole, 2-pyrazoline, pyrazolidine, 1H-1,2,3-triazole, 1H-1,2,4-triazole, 4H-1,2,4-triazole, thiazole, isothiazole, 2-thiazoline, thiazolidine, oxadole, isoxazole, 1,2,5-oxadiazole, pyridine, pyperidine, pyperadine, pyridazine, pyrimidine, pyradine, 2H-1,3-thiadine, 6H-1,3-thiadine, 2H-1,4-thiadine, 4H-1,4-thiadine, 4H-1,4-oxadine, azepin, 1,n-diazepin (n = 2 - 4) and azocine can be specifically exemplified. Moreover, as for the substituted group to be bound to the heterocycle formed with the atom group represented by C, the same substituted groups to be bound to the above R¹, R² or R³ can be exemplified, and it can be a group condensing to a heterocycle to form an ortho-condensed group; and as for an ortho-condensed ring formed with a heterocycle, specifically, indole, indoline, isoindole, isoindoline, indazole, 2H-indazole, benzoimidazole, benzothiazole, benzothiazoline, benzoxazole, 1,2-benzoisoxazole, quinoline, isoquinoline, cinnoline, quinazoline, quinoxaline, phtalazine, 1,8-naphthyridine can be exemplified.

Moreover, in the compound shown by general formulae (II) and (III) of the present invention, Q represents a pharmaceutically acceptable anion, and it can be any anion as long as it is pharmaceutically acceptable, including halogen ion, sulfonate ion, sulfamate ion, and hydroxide ion. For instance, as for halogen ion, chloride ion, bromide ion and iodide ion can be exemplified; as for sulfonate ion, aliphatic and aromatic sulfomate ion such as methanesulfonate ion, ethansulfonate ion, trifluoromethanesulfonate ion, p-toluene sulfonate ion, naphthalene sulfonate ion, 2-hydroxyethane sulfonate ion can be specifically exemplified. As for sulfamate ion, cyclohexane sufamate ion can be exemplified, further including sulfate ion such as methylsulfate ion and ethylsulfate ion; hydrogen sulfate ion, borate ion, alkyl and dialkyl phosphate ion, carboxylate ion and carbonate ion. Specific and preferable examples of pharmaceutically acceptable anion include chloride ion, bromide ion, iodide ion, acetate ion, propionate ion, valerate ion, citrate ion, maleate ion, fumarate ion, lactate ion, succinate ion, tartrate ion, benzoate ion, methanesulfonate ion, ethanesulfonate ion, p-toluene sulfonate ion and hydroxide ion.

As for the above compound shown by general formula (II), examples include, 2-[(3-methylthiazolilidene)methyl]-1-methylpyridinium salts, 2-[{3-(2-propenyl)thiazolilidene}methyl]-1-methylpyridinium salts, 2-[(3-methylthiazolilidene)methyl]-1-methylquinolinium salts, 2-[{3-(2-propenyl)thiazolilidene}methyl]-1-methylquinoliniu m salts, and preferably 2-[(3-methyl-2(3H)-benzothiazolylidene)methyl]-1-methylpyri dinium=p-toluenesulfonate [formula (V) (a)] or 2-[(4-oxo-3-ethyl-2-thiazolidinylidene)methyl]-3-ethylbenzo thiazolium=p-toluenesulfonate [formula (V) (b)] can be exemplified.

Further, as for the compounds shown by general formula (II), rhodacine dye compounds shown by formulae (I) (a) to (d) or formulae (IV) (a) to (m) can be preferably and specifically exemplified. In other words, examples include: 2-[{5-(3-methyl-2(3H)-benzothiazolylidene)-4-oxo-3-(2-propenyl)-2-thiazolidinylidene}methyl]-1-methylpyridinium=p-toluene sulfonate [formula (IV) (a)], 4-[{5-(3-methyl-2(3H)-benzothiazolylidene)-4-oxo-3-ethyl-2-thiazolidinylidene}methyl]-1-methylquinolium=p-toluene sulfonate [formula (IV) (b)], 2-[{5-(3-methyl-2(3H)-benzothiazolylidene)-4-oxo-3-ethyl}-2-thiazolidinylidene]methyl]methyl-1-methylquinolium=iodide [formula (IV) (c)], 2-[{5-3-methyl-2(1H)-quinolinidene-4-oxo-3-ethyl}-2-thiazolidinylidene]methyl]-3-methylbenzothiazolium=p-toluenesulfonate [formula (IV) (d)], 2-[[5-(5'-(3''-methyl-2'' (3''H)-benzothiazolylidene)-4'-oxo-3'-ethyl-2'-thiazolidinylidene}-4-oxo-3-ethyl-2-thiazolidinylidene]methyl]-3-ethylbenzothiazolium=bromide [formula (IV) (e)], 2-[{5-(3-methyl-2(3H)-benzothiazolylidene)-4-oxo-3-benzyl-2-thiazolidinylidene}methyl]-1-methylpyridinium=p-toluenesul fonate [formula (I) (a)], 2-[{5-(5-chloro-3-methyl-2(3H)-benzothiazolylidene)-4-oxo-3-ethyl-2-thiazolidinylidene)methyl]-1-methylpyridinium=p-toluenesulfonate[formula(IV)(f)],2-[{5-(3-methyl-2(3H)-benzothiazolylidene)-4-oxo-3-ethyl-2-thiazolidinylidene}methyl]-3-methylbenzo[e]benzothiazolium=p-toluenesulfonate[formula(IV)(g)], 2-[{5-(5-chloro-3-methyl-2(3H)-benzothiazolylidene)-4-oxo-3-(2-propenyl)-2-thiazolidinylidene}methyl]-1-methylpyridinium=p-toluenesulfonate[formula(IV)(h)], 2-[{5-(3-methyl-2(3H)-benzothiazolylidene)-4-oxo-3-ethyl-2-thiazolidinylidene}methyl]-1-ethylpyridinium=chloride[formula(IV)(i)]4-[(5-(3-methyl-2(3H)-benzothiazolylidene)-4-oxo-3-ethyl-2-thiazolidinylidene}methyl]-1-methylpyridinium=p-toluenesulfonate[formula(IV)(j)], 4-[{5-(3-methyl-2(3H)-benzothiazolylidene)-4-oxo-3-ethyl-2-thiazolidinylidene}methyl]-1-n-butylpyridinium=bromide[formula(I)(b)], 2-[{5-(3-methyl-2(3H)-benzothiazolylidene)-4-oxo-3-ethyl-2-thiazolidinylidene}methyl]-1-n-butylpyridinium=iodide[formula(I)(c)],1-(p-carboxyphenyl)methyl-2-[(5-(3-methyl-2(3H)-benzothiazolylidene)-4-oxo-3-ethyl-2-thiazolidinylidene)methyl]pyridinium=bromide[formula(I)(d)], 2-[{5-(3-methyl-2(3H)-benzothiazolylidene)-4-oxo-3-(2-tetrahydrofuranyl)methyl-2-thiazolidinylidene}methyl]-1-methylbenzothiazolium=p-toluenesulfonate[formula(IV)(k)], 2-[{5-(3-methyl-2(3H)-benzobenzo[e]thiazolilidene)-4-oxo-3-(2-tetrahydrofuranyl)methyl-2-thiazolidinylidene}methyl]-1-methylpyridinium=p-toluenesulfonate[formula(IV)(1)], 2-[(5-(3-methyl-2(3H)-benzobenzo[e]thiazolilidene)-4-oxo-3-ethyl-2-thiazolidinylidene)methyl]-1-methylbenzothiazolium-p-toluenesulfonate[formula(IV) (m)].

The method for preparing a rhodacyanine dye compound shown by such formula (IV) is not particularly limited, and can be performed according to a well-known method. For example, it can be synthesized by a method described in Journal of Medicinal Chemistry, vol. 45, pp. 995-998, 2002. The intended rhodacyanine dye compound shown by general formula (III) can be obtained for example by the following steps: 1) a step of suspending a mixture of thiobenzothiazole derivative and alkyl sulfonate etc., and 2-thioxo-4-thiazolidinone into a solvent, and allowing the mixture to react in the presence of amine etc. to bind the two thiazolidine derivatives; 2) a step of suspending the obtained compound with methylsulfonate etc. into a solvent, and heating the mixture to obtain a conjugate acid; 3) a step of heating and reacting the conjugate acid and the pyridium compound etc. , in the presence of amine etc. Further, a combinatorial method to obtain various compound collections can be also used, and for example, a method for obtaining m × n x r types of rhodacyanine dye compounds, by reacting m types of heterocyclic compounds containing nitrogen with n types of rhodanine ring compounds in m x n reaction containers with a filtrating-means in the presence of solvent such as acetonitrile, in different combinations to remove liquid phase; thiomethylating the resulting m x n merothianine dye compounds; reacting in the presence of solvent such as acetonitrile, with r types of specific heterocyclic compounds containing nitrogen, in different combinations may be used.

In case of using the compound shown by the above general formulae (II), (III) to prevent, suppress, and treat infections by leishmania protozea, the administration can be performed orally, by subcutaneous or intravenous injections, topically, etc. Further, as for formulations, generally, oral agents such as powder agent, tablet, fine granule agent, pill, capsule, granule prepared with the use of pharmaceutically acceptable carriers, excipients, other additives, and parental agents such as instillation, injection, and suppository can be exemplified. As for pharmaceutically acceptable carriers, excipients or other additives, examples include glucose, lactose, gelatin, mannitol, starch paste, magnesium trisilicate, corn starch, keratin and colloidal silica. Adjuvants such as stabilizer, expander, coloring agent, and aromatizing agent can be also included. Each of these formulations can be prepared by preparation methods known and common to those skilled in the art. Moreover, the dose per day varies according to patient's symptoms, body weight, age, sex, etc. and cannot be determined uniformly, but it is preferable to administer the compound of the present invention in an amount of 0.1 to 1000 mg per day, and preferably 1 to 600 mg per day for an adult.

### (Examples)

In the following, the present invention will be explained further in detail by referring to the examples, but the technical scope of the present invention will not be limited to these examples.

### Example 1: Synthesis of 2-[{5-(3-methyl-2(3H)-benzothiazolylidene)-4-oxo-3-benzyl-2 -thiazolidinylidene}methyl]-1-methylpyridinium=p-toluenesul fonate [formula (I)]

### (1) Synthesis of 5-(3-methyl-2(3H)-benzothiazolylidene)-2-thioxo-3-benzyl-4-thiazolidinone

To a mixture of 373 mg of 3-methyl-2-methylthio benzothiazolium=p-toluenesulfonate and 228 mg of 3-benzyl-2-thioxo-4-thiazolidinone, 3.6 mL of acetonitrile was added to obtain a suspending solution. The solution was cooled down to 10°C. To this mixture, an amount of 0.22 mL of triethylamine was added dropwise at 10°C, and the resultant was stirred for 3 hours at 10°C. The obtained precipitate was aspirated and filtrated, washed with methanol, and then dried. The above compound was thus obtained in an amount of 354 mg. The yield was 94%.

### (2) Synthesis of 4,5-dihydro-5-(3-methyl-2(3H)-benzothiazolylidene)-2-methyl thio-3-benzyl-4-oxothiazolium=p-toluenesulfonate

To a mixture of 314 mg of 5-(3-methyl-2(3H)-benzothiazolylidene)-2-thioxo-3-benzyl-4-thiazolidinone and 468 mg of p-toluenemethylsulfonate, 0.3 mL of dimethylformanide was added to obtain a suspension solution. The solution was stirred for 3.5 hours at 130°C. The obtained mixture was cooled down to room temperature and then acetone was added. The precipitate was aspirated and filtrated, washed with cooled acetone, and then dried. The above compound was thus obtained in an amount of 419 mg. The yield was 89%.

### (3) Synthesis of 2-[{5-(3-methyl-2(3H)-benzothiazolylidene)-4-oxo-3-benzyl-2 -thiazolidinylidene}methyl]-1-methylpyridinium=p-toluenesul fonate

An amount of 200 mg of 4,5-dihydro-5-(3-methyl-2(3H)-benzothiazolylidene)-2-methyl thio-3-ethyl-4-oxothiazolium=p-toluenesulfonate and 100 mg of 1,2 dimethylpyridinium=p-toluenesulfonate were suspended into 1.8 mL of acetonitrile, and heated to 70°C. To this mixture, 0.16 mL of triethylamine was added dropwise at 70°C, and the resultant was stirred for 1 hour. The resultant was cooled down to room temperature, ethyl acetate was added, and the precipitate was aspirated and filtrated. The crude crystals were washed with ethyl acetate, and then dried. The above compound was thus obtained in an amount of 62.6 mg. The yield was 27%.
mp 211-216°C; 1H-NMR (DMSO-d6)d: 2.28 (3H, s), 3.86 (3H, s), 4.08 (3H, s), 5.30 (2H, s), 5.85 (1H, s), 7.11 (2H, d, J = 8.0 Hz), 7.30-7.53 (10H, m), 7.64 (1H, d, J = 8.2 Hz), 7.90 (1H, d, J = 7.1 Hz), 7.97 (1H, d, J = 8.2 Hz), 8.20 (1H, t, J = 7.9 Hz), 8.59 (1H, d, J = 6.6 Hz); Anal Calcd for C32H29N3O4S3· 1.4H2O: C, 59.96; H, 5.00; N, 6.56. Found: C, 60.23; H, 5.27; N, 6.50.

### Example 2: Synthesis of 1-butyl-4-[3-ethyl-{5-(3-methyl-2(3H)-benzothiazolylidene)-4-oxo-2-thiazolidinylidene}methyl]pyridinium=bromide [formula (I) (b)]

An amount of 300 mg of 3-ethyl-4,5-dihydro-5-(3-methyl-2(3H)-benzothiazolylidene)-2-methylthio-4-oxothiazolium=p-toluenesulfonate and 115.8 mg of 4-butyl-2-methylpyridinium=bromide were suspended into 2.5 mL of acetonitrile, and heated to 70°C. To this mixture, 0.21 mL of triehtylamine was added dropwise at 70°C, and stirred for 30 min. After cooling down the resultant to room temperature, ethyl acetate was added and the precipitate was aspirated and filtrated. The crude crystals were washed with ethyl acetate and then dried. The above compound was thus obtained in an amount of 155.4 mg. The yield was 52%.
m.p. 299-300°C; IR (KBr) cm-1: 1179, 1382, 1496, 1533, 1542, 1639; 1H-NMR (300 MHz, DMSO-d6) δ: 0.91 (3H, t, J = 7.3 Hz), 1.21 (3H, t, J = 7.1 Hz), 1.19-1.31 (2H, m), 1.81 (2H, q, J = 7.3 Hz), 4.00 (2H, q, J = 7.1 Hz), 4.07 (3H, s), 4.31 (2H, t, J = 7.3 Hz), 6.34 (1H, s), 7.29 (1H, dd, J = 7.3, 7.8 Hz), 7.48 (1H, dd, J = 7.4, 7.8 Hz), 7.61 (3H, m), 7.85 (1H, d, J = 7.3 Hz), 8.47 (2H, d, J = 7.1 Hz); Anal Calcd for C23H26BrN3OS2: C, 54.76; H, 5.19; N, 8.33. Found; C, 54.48; H, 4.97; N8.08.

### Example 3: Synthesis of 1-butyl-2-[3-ethyl-{5-(3-methyl-2(3H)-benzothiazolylidene)-4-oxo-2-thiazolidinylidene}methyl]pyridinium=iodide [formula (I) (c)]

An amount of 642.7 mg of 3-ethyl-4,5-dihydro-5-(3-methyl-2(3H)-benzothiazolylidene)-2-methylthio-4-oxothiazolium=p-toluenesulfonate and 299.3 mg of 1-butyl-2-methylpyridinium=iodide were suspended into 5.0 mL of acetonitrile, and heated to 70°C. To this mixture, 0.45 mL of triethylamine was added dropwise at 70°C, and stirred for 1. 5 hours. After cooling down the mixture to room temperature, ethyl acetate was added and the precipitate was aspirated and filtrated. The crude crystals were washed with ethyl acetate and then dried. The above compound was thus obtained in an amount of 344.5 mg. The yield was 58%.
m.p. 237-242°C; 1H-NMR (300 MHz, DMSO-d6) d: 0.93 (3H, t, J = 7.4 Hz), 1.23 (3H, t, J = 7.4 Hz), 1.38 (2H, tq, J = 7.4, 7.0 Hz), 1.79 (2H, tt, J = 7.1, 7.0 Hz), 4.02 (s, 3H), 4.09 (2H, q, J = 7.4 Hz), 4.57 (2H, t, J = 7.1 Hz), 5.97 (1H, s), 7.28 (1H, t, J=7.1 Hz), 7.437.50 (2H, m), 7.59 (1H, d, J=8.0 Hz), 7.85 (1H, d, J = 7.1 Hz), 8.06 (1H, d, J = 7.7 Hz), 8.27 (1H, t, J = 7.1 Hz), 8.67 (1H, d, J = 5.2 Hz); Anal Calcd for C23H26IN3OS2 · 1H20: C, 48.51; H, 4.96; N, 7.38. Found: C, 48.67; H, 4.91; N, 7.36.

### Example 4: Synthesis of 1-(p-carboxyphenyl)methyl-2-[3-ethyl-{5-(3-methyl-2(3H)-ben zothiazolylidene)-4-oxo-2-thiazolidinylidene}methyl]pyridin ium=bromide [formula (I) (d)]

### (1) Synthesis of 1-(p-carboxyphenyl)methyl-2-[3-ethyl-{5-(3-methyl-2(3H)-ben zothiazolylidene)-4-oxo-2-thiazolidinylidene}methyl]pyridin ium=triethylammonium

An amount of 573.3 mg of 3-ethyl-4,5-dihydro-5-(3-methyl-2(3H)-benzothiazolylidene)-2-methylthio-4-oxothiazolium=p-toluenesulfonate and 356.2 mg of 1-(p-carboxyphenyl)methyl-2-methylpyridinium=bromide were suspended into 5.8 mL of acetonitrile, and heated to 70°C. To this mixture, 0.5 mL of triethylamine was added dropwise at 70°C, and stirred for 4 hours. After cooling down the resultant to room temperature, ethyl acetate was added and the precipitate was aspirated and filtrated. The crude crystals were dissolved in methanol, and crystals were deposited with ethyl acetate. Then, the crystals were washed with ethyl acetate and dried. The above compound was thus obtained in an amount of 358.1 mg. The yield was 45%.

### (2) Synthesis of 1-(p-carboxyphenyl)methyl-2-[3-ethyl-{5-(3-methyl-2(3H)-ben zothiazolylidene)-4-oxo-2-thiazolidinylidene}methyl]pyridin ium=bromide

The above compound was suspended into 5.0 mL of methanol, and appropriate amount of the hydrogen bromide ethanol solution was added at 0°C. After stirring for 30 min, ethyl acetate was added, and the precipitate was aspirated and filtrated. The crude crystals were washed with ethyl acetate and then dried. The above compound was thus obtained in an amount of 298.6 mg. The yield was 100%.
m.p. >300°C; IR (KBr) cm-1: 1171, 1650, 1495, 1614, 1651, 3430; 1H-NMR
(300 MHz, DMSO-d6) d: 0.78 (3H, t, J = 6.9 Hz), 3.86 (2H, q, J = 6.9 Hz), 4.03 (3H, s), 5.74 (1H, s), 5.93 (2H, s), 7.28 (2H, d, J=7.8 Hz), 7.28-7.33 (1H, m) , 7.45-7.51 (2H, m), 7.62 (1H, d, J = 8.8 Hz), 7.86 (1H, d, J = 8.5 Hz), 7.95 (2H, d, J = 7.8 Hz), 8.06 (1H, d, J = 8.5 Hz), 8.33 (1H, dd, J = 8.0, 8.0 Hz), 8.83 (1H, d, J = 6.3 Hz).

### Example 5: Synthesis of 2-[(3-methyl-2(3H)-benzothiazolylidene)methyl]-1-methylpyri dinium=p-toluenesulfonate [formula (V)]

An amount of 326.1 mg of 3-methyl-2-methylthio benzothiazolium=p-toluenesulfonate and 278.1 mg of 1,2-dimethylpyridinium=p-toluenesulfonate were suspended into 4.4 mL of acetonitrile, and heated to 70°C. To this mixture, 0.37 mL of triethylamine was added dropwise at 70°C, and stirred for 1 hour. After cooling down the mixture to room temperature, ethyl acetate was added and the precipitate was aspirated and filtrated. The crude crystals were washed with ethyl acetate and then dried. The above compound was thus obtained in an amount of 166.1 mg. The yield was 44%.
m.p. 248-254°C; IR (KBr) cm-1: 1171, 1200, 1380, 1456, 1494, 1532, 1632; 1H-NMR (300 MHz, DMSO-d6) δ: 2.28 (3H, s), 3.78 (3H, s), 4.07 (3H, s), 5.83 (1H, s), 7.11 (2H, d, J = 8.0 Hz), 7.22-7.27 (1H, m) , 7.29-7.34 (1H, m), 7.62 (2H, d, J = 8.0 Hz), 7. 50-7. 56 (1H, m), 7.62 (1H, d, J = 8.0 Hz), 7.89-7.92 (2H, m) , 8.16 (1H, dd, J = 7.4, 7.4 Hz), 8.53 (1H, d, J = 6.3 Hz); Anal Calcd for C22H22N2O3S2·0.2H2O: C, 61.43; H, 5.25; N, 6.51. Found: C, 61.23; H, 5.24; N, 6.88

### Example 6: Synthesis of 2-[{5-(3-methyl-2(3H)-benzothiazolylidene)-4-oxo-3-(2-prope nyl)-2-thiazolidinylidene}methyl]-1-methylpyridinium=p-tolu enesulfonate [formula (IV) (a)]

An amount of 4.0 g of 2-methylthiobenzothiazole and 5.5mL of anisole solution containing 5.0 mL of p-toluenemethylsulfonate were stirred for 1.5 hours at 130°C. After cooling down the mixture to room temperature, 74 mL of acetonitrile was added, and 3.83 g of 3-(2-propenyl)-2-thioxo-4-thiazolidinone was further added. After cooling down the mixture to 0°C, 4.9 mL of triethylamine was gradually dropped and the mixture was stirred for 1 hour at 0°C. The obtained precipitate was aspirated and filtrated, washed with acetonitrile, and 5.51 g of crude crystals was obtained. To the mixture of 2.1 g of the crude crystals and 9.0 mL of p-toluenemethylsulfonate, 7.5 mL of dimethylformamide was added to make a suspension solution. The solution was stirred for 1.5 hours at 120°C, cooled down to room temperature, and then acetone was added. The precipitate was aspirated and filtrated. The resultant was washed with acetone and then dried, to obtain 8.64 g of crude crystals. The mixture of 8.64 g of the crude crystals, 4.71 g of 1-methyl-4-methylpyridinium=p-toluenesulfonate and 85.0 mL of acetonitrile was stirred. To this mixture, 7.0 mL of triethylamine was added dropwise at 70°C, and stirred for 1 hour at 70°C. The obtained compound was cooled down to room temperature, and then ethyl acetate was added. The precipitate was aspirated and filtrated, and washed with cold ethyl acetate. The crude crystals were recrystallized from the mixed solvent of methanol/ethyl acetate, to obtain 3.94 g of 2-[{5-(3-methyl-2(3H)-benzothiazolylidene)-4-oxo-3-(2-prope nyl)-2-thiazolidinylidene}methyl]-1-methylpyridinium=p-tolu enesulfonate. The yield was 32%.
m.p. 249-251°C; IR (KBr) cm-1: 1038, 1189, 1057, 1539, 1636, 3471; 1H-NMR (300 MHz, DMSO-d6 ) :δ 2.27 (3H, s), 4.04 (6H, s), 4.69 (2H, d, J=4.9 Hz), 5.27-5.33 (2H, m), 5.81-5.92 (1H, m) , 5.85 (1H, s), 7.09 (2H, d,J = 7.7 Hz), 7.28 (1H, dd, J = 7.4, 7.7 Hz), 7.40 (1H, dd, J = 6.5, 7.6Hz), 7.45-7.49 (3H, m), 7.60 (1H, d, J = 8.2 Hz), 7.85 (1H, d, J = 7.7Hz), 7.98 (1H, d, J = 8.2 Hz), 8.25 (1H, dd, J = 7.6, 8.2 Hz), 8.63 (1H, d, J = 6.5 Hz); Anal Calcd for C29H27N3O4S3: C, 59.45; H, 4.81; N, 7.43: Found: C, 59.25, H, 4.80; N, 7.29.

### Example 7: Synthesis of 2-[{5-(3-methyl-2(3H)-benzothiazolylidene)-4-oxo-3-ethyl-2-thiazolidinylidene}methyl]-1-methylquinolinium=iodide [formula (IV)(c)]

### (1) Synthesis of 5-(3-methyl-2(3H)-benzothiazolylidene)-2-thioxo-3-ethyl-4-t hiazolidinone

Anisole solution in an amount of 4.14 mL with 2.98 g of 2-methylthiobenzothiazole and 3.74 mL of p-toluenemethylsulfonate was stirred for 4 hours at 120°C. Then, the mixture was cooled down to room temperature, 60 mL of acetenitrile was added, and further stirred for 15 min at room temperature. Then, to this mixture of 2.67 g of 2-thioxo-3-ethyl-4-thiazolidinone, 3.6 mL of acetonitrile was added to make a suspension solution and cooled down to 10°C. To this mixture, 3.6 mL of triethylamine was added dropwise at 10°C and the resultant was stirred for 4 hours at 10°C. The obtained precipitate was aspirated and filtrated, washed with methanol and then dried. The above compound was thus obtained in an amount of 4.52 mg. The yield was 89%.

### (2) Synthethis of 4,5-dihydro-5-(3-methyl-2(3H)-benzothiazolylidene)-2-methyl thio-3-ethyl-4-oxothiazolium=p-toluenesulfonate

To a mixture of 2.1 g of 5-(3-methyl-2(3H)-benzothiazolylidene)-2-thioxo-3-ethyl-4-t hiazolidinone and 3.85 g of p-toluenemethylsulfonate, 2.3 mL of dimethylformamide was added to make a suspension solution. The solution was stirred for 2.5 hours at 130°C. The obtained mixture was cooled down to room temperature and acetone was added. The precipitate was aspirated and filtrated, washed with cold acetone and then dried. The above compound was thus obtained in an amount of 3.0 g. The yield was 90%.

### (3) Synthethis of 2-[{5-(3-methyl-2(3H)-benzothiazolylidene)-3-ethyl-4-oxo-2-thiazolidinylidene}methyl]-1-methylquinolinium=iodide

An amount of 114.3 mg of 4,5-dihydro-5-(3-methyl-2(3H)-benzothiazolylidene)-2-methyl thio-3-ethyl-4-oxothiazolium=p-toluenesulfonate and 66.1 mg of 1,2 dimethyl quinolizinylium=iodide was suspended to 1.2 mL of acetonitrile, and heated to 70°C. To this mixture, 0.10 mL of triethylamine was added at 70°C, and stirred for 1 hour. After cooling down the mixture to room temperature, ethyl acetate was added and the precipitate was aspirated and filtrated. The crude crystals were washed with ethyl acetate and then dried. The above compound was thus obtained in an amount of 114.4 mg. The yield was 88%.
mp 283-285°C; IR (KBr) cm-1: 1177, 1353, 1500, 1533, 1608, 1640; 1H-N
MR (DMSO-d6)δ: 1.30 (3H, t, J = 7.1 Hz) , 4.11 (3H, s) , 4.21-4.25 (5H,m), 6.23 (1H, s), 7.34 (1H, dd, J=7.4, 7.7 Hz), 7.52 (1H, dd, J = 7.4, 8.8 Hz), 7.68-7.74 (2H, m), 7.93 (1H, d, J = 8.0 Hz), 7.99 (1H, dd, J = 7.4, 8.5 Hz), 8.14 (1H, d, J = 7.7 Hz), 8.21 (1H, d, J = 8.8 Hz), 8.25 (1H, d, J = 8.5 Hz), 8.58 (1H, d, J = 7.1 Hz); Anal Calcd for C24H22IN3OS2 · 0.5H20: C, 50.71; H, 4.08; N, 7.39. Found: C, 50.76; H, 4.02; N, 7.32.

### Example 8: Synthesis of 2-[{5-(3-methyl-2(3H)-benzothiazolylidene)-4-oxo-3-ethyl-2-thiazolidinylidene}methyl]-1-methylquinolinium=iodide [formula (IV) (g)]

An amount of 340.8 mg of 4,5-dihydro-5-(3-methyl-2(3H)-benzothiazolylidene)-2-methyl thio-3-ethyl-4-oxothiazolium=p-toluenesulfonate synthesized by the operation of (1) and (2) of Example 4, and 223.6 mg of 2,3-dimethylnaphth[1,2-d]thiazolium=p-toluenesulfonate were suspended into 2.8 mL of acetonitrile, and the mixture was heated to 70°C. To this mixture, 0.11 mL of triethylamine was added dropwise at 70°C, and stirred for 1 hour. After cooling down the mixture to room temperature, ethyl acetate was added, and the precipitate was aspirated and filtrated. The crude crystals were washed with ethyl acetate and dried. The above compound was thus obtained in an amount of 147.6 mg. The yield was 86%.
m.p. >300°C; IR (KBr) cm-1: 1200, 1371, 1467, 1503, 1539, 1649, 3422;
1H-NMR (300 MHz, DMSO-d6) δ: 1.30 (3H, t, J = 7.1 Hz), 2.27 (3H, s), 4.12 (3H, s), 4.28 (2H, q, J = 7.1 Hz), 4.49 (3H, s), 6.78 (1H, s), 7.09 (2H, d, J = 8.0 Hz), 7.30 (1H, dd, J = 7.4, 7.7 Hz), 7.45-7.52 (3H, m), 7.45-7.79 (3H, m), 7.92 (1H, d, J = 7.7 Hz), 8.04 (1H, d, J = 8.8 Hz), 8.14 (1H, d, J = 8.2 Hz), 8.24 (1H, d, J = 8.8 Hz), 8.81 (1H, d, J = 8.5 Hz); Anal Calcd for C33H29N3O4S4·1.4H2O: C, 57.86; H, 4.68; N, 6.13. Found: C, 58.05, H, 4.80, N, 5.84.

### Example 9: Leishmania protozoa proliferation inhibition assay

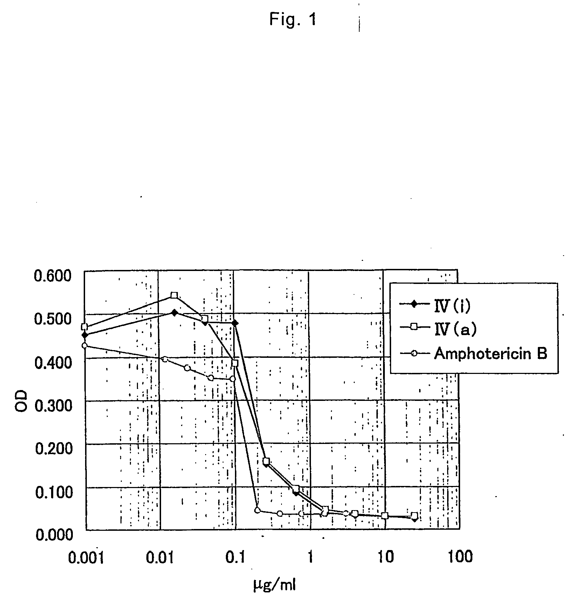

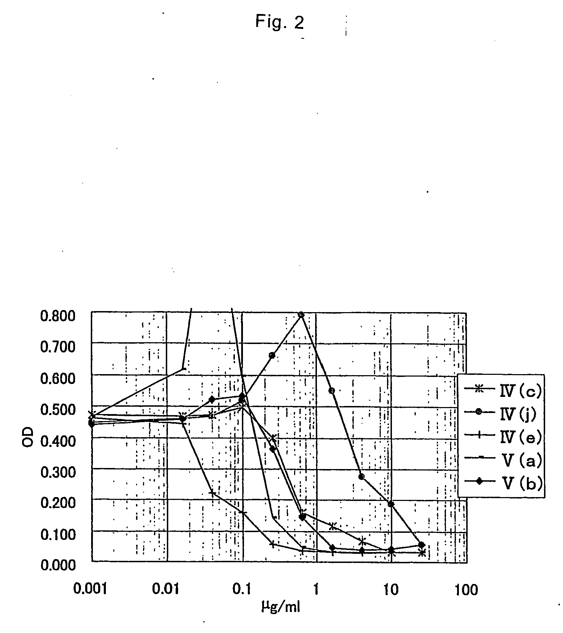

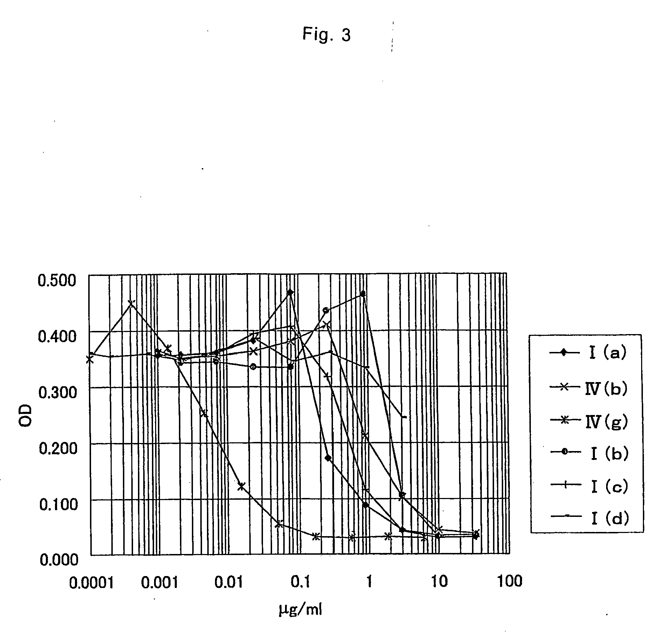

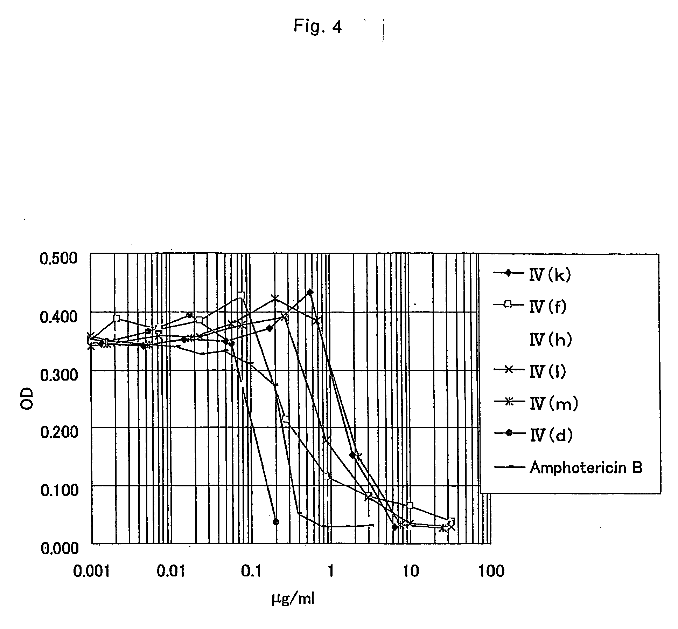

After dissolving each of the obtained compounds in dimethylsulfoxide (hereinafter referred to as DMSO), the resultant was diluted by 9 steps in Medium 199 medium (Invitrogen) to the indicated concentration, filtrated with a membrane filter, and each of the filtration was made as a sample solution. An amount of 50 µL each of the above sample solutions, and 50 µL of Leishmania (L. major) culture solution prepared so that the final concentration become 2 x 10⁻⁵/mL were inoculated to 96-well microtiter plate, respectively, and the total amount of the culture solution was made as 100 µL. By keeping the solution at 27°C, incubation was performed under 5% CO₂, for 48 hours. Then, TetraColor ONE (tradename: Seikagaku Corporation) reagent was added thereto and further incubated for 6 hours. Then, the absorbance at a wavelength of 450 nm was measured for the 96-well microtiter plate with a microplate reader, the wave length of 630 nm was made as reference wave length, and in order to exclude background effect, the absorbance at 630 nm was excluded from the one at 450 nm, and the OD level was calculated as a level to indicate the number of viable Leishmania protozoa. The assay was performed by three wells at a time for each concentration of each sample, and the mean level was made as OD level. The calculated OD levels are shown in Tables 1 to 3. According to these results, a graph showing the relation between concentration of each sample and OD level, that is the relation between the concentration of the sample and the number of viable Leishmania protozoa was prepared. The results are shown in Figs. 1 to 4. Amphotericin B was used as positive control.

**(Table 1)**

| | | | | | | | | | | |
|---|---|---|---|---|---|---|---|---|---|---|
| mg/ml | 25 | 10 | 4 | 1.6 | 0.64 | 0.256 | 0.1024 | 0.04096 | 0.016384 | 0.001 |
| IV (i) | 0.026 | 0.030 | 0.033 | 0.039 | 0.086 | 0.153 | 0.478 | 0.482 | 0.504 | 0.451 |
| IV (a) | 0.032 | 0.031 | 0.035 | 0.047 | 0.095 | 0.157 | 0.385 | 0.488 | 0.541 | 0.470 |
| IV(c) | 0.032 | 0.032 | 0.069 | 0.117 | 0.158 | 0.398 | 0.496 | 0.474 | 0.470 | 0.472 |
| IV (j) | 0.057 | 0.188 | 0.275 | 0.551 | 0.793 | 0.661 | 0.518 | 0.468 | 0.462 | 0.446 |
| IV (e) | 0.033 | 0.032 | 0.030 | 0.030 | 0.035 | 0.056 | 0.159 | 0.220 | 0.445 | 0.463 |
| V (a) | 0.032 | 0.031 | 0.029 | 0.032 | 0.046 | 0.139 | 0.596 | 1.237 | 0.615 | 0.464 |
| V (b) | 0.056 | 0.042 | 0.040 | 0.047 | 0.145 | 0.363 | 0.533 | 0.520 | 0.458 | 0.441 |
| mg/ml | 3.1 | 1.55 | 0.775 | 0.3875 | 0.19375 | 0.096875 | 0.0484375 | 0.0242188 | 0.0121094 | 0.001 |
| Ampho tericin B | 0.034 | 0.036 | 0.036 | 0.036 | 0.044 | 0.349 | 0.352 | 0.373 | 0.394 | 0.428 |

**(Table 2)**

| | | | | | | | | | | |
|---|---|---|---|---|---|---|---|---|---|---|
| mg/ml | 3.1 | 0.93 | 0.279 | 0.084 | 0.025 | 0.008 | 0.002 | 0.0007 | 2E-04 | 0.0001 |
| I (a) | 0.035 | 0.034 | 0.043 | 0.088 | 0.172 | 0.469 | 0.383 | 0.361 | 0.356 | 0.356 |
| IV (b) | 0.037 | 0.044 | 0.102 | 0.211 | 0.409 | 0.380 | 0.362 | 0.354 | 0.351 | 0.361 |
| IV (g) | 0.029 | 0.031 | 0.030 | 0.032 | 0.055 | 0.121 | 0.253 | 0.368 | 0.448 | 0.352 |
| I (b) | 0.032 | 0.032 | 0.106 | 0.465 | 0.435 | 0.335 | 0.335 | 0.345 | 0.343 | 0.363 |
| I (c) | 0.031 | 0.029 | 0.042 | 0.117 | 0.317 | 0.407 | 0.394 | 0.360 | 0.350 | 0.365 |
| I (d) | 0.242 | 0.333 | 0.360 | 0.346 | 0.384 | 0.364 | 0.348 | 0.359 | 0.356 | 0.360 |

**(Table 3)**

| | | | | | | | | | | |
|---|---|---|---|---|---|---|---|---|---|---|
| mg/ml | 33 | 9.9 | 2.97 | 0.891 | 0.2673 | 0.08019 | 0.02406 | 0.0072171 | 0.002165 | 0.001 |
| IV (k) | 0.029 | 0.154 | 0.434 | 0.371 | 0.348 | 0.351 | 0.341 | 0.344 | 0.356 | 0.338 |
| IV (f) | 0.038 | 0.064 | 0.081 | 0.116 | 0.214 | 0.428 | 0.384 | 0.369 | 0.388 | 0.352 |
| IV (h) | 0.034 | 0.029 | 0.039 | 0.079 | 0.140 | 0.277 | 0.423 | 0.375 | 0.368 | 0.352 |
| IV (1) | 0.029 | 0.035 | 0.080 | 0.178 | 0.391 | 0.377 | 0.357 | 0.358 | 0.346 | 0.357 |
| IV (m) | 0.027 | 0.033 | 0.149 | 0.385 | 0.422 | 0.379 | 0.354 | 0.342 | 0.344 | 0.340 |
| IV (d) | 0.035 | 0.344 | 0.395 | 0.365 | 0.348 | 0.359 | 0.341 | 0.339 | 0.357 | 0.346 |
| Ampho tericin B | 0.030 | 0.029 | 0.029 | 0.051 | 0.273 | 0.311 | 0.331 | 0.325 | 0.338 | 0.351 |

Further, from the graphs of Figs. 1 to 4 showing the relation between the concentration of each sample and the number of viable leishmania protozoa, IC₅₀ level showing concentration inhibiting 50% cell proliferation of leishmania protozoa in each sample (g/ml) was calculated. The results are shown in Table 4. Inhibiting effect against leishmaia protozoa for each sample was similar or more compared with that of Amtephotericin B.

**(Table 4)**

| compound | Concentration inhibiting 50% leishmania proliferation (M) |
|---|---|
| V (a) | 4.9 x 10⁻⁷ |
| V (b) | 1.2 x 10⁻⁶ |
| IV (a) | 3.2 x 10⁻⁷ |
| N (b) | 1.8 x 10⁻⁶ |
| N (c) | 8.2 x 10⁻⁷ |
| N (d) | 1.8 x 10⁻⁷ |
| N (e) | 6.1 x 10⁻⁸ |
| I (a) | 4.1 x 10⁻⁷ |
| IV (f) | 5.4 x10⁻⁷ |
| N (g) | 1.2 x 10⁻⁸ |
| IV (h) | 2.7 × 10⁻⁷ |
| N (i) | 4.6 × 10⁻⁷ |
| IV (j) | 1.0 × 10⁻⁵ |
| I (b) | 3.7 x 10⁻⁶ |
| I (c) | 1.0 x 10⁻⁶ |
| I (d) | 5.3 x 10⁻⁶ |
| N (k) | 2.6 x 10⁻⁶ |
| IV (1) | 1.3 x 10⁻⁶ |
| N (m) | 2.7 x 10⁻⁶ |
| Amphotericin B | 1.4 x 10⁻⁷ |

### Industrial Applicability

The anti-leishmania agent of the present invention has an excellent anti-leishmania activity with fewer side effects, and as it is easy to manufacture, it is excellent as being versatile as an anti-leishmania agent. Particularly, the one shown by general formula (III) containing rhodacyanine dye compound as an active ingredient has an excellent anti-leishmania activity, is low-toxic and easy to manufacture, and it is extremely useful as an anti-leishmania agent.

## Claims

1. A compound shown by formula (I) (a).

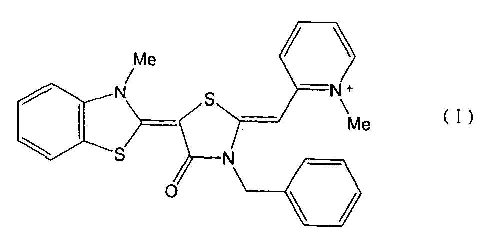

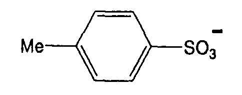

2. A compound shown by formula (I) (b).

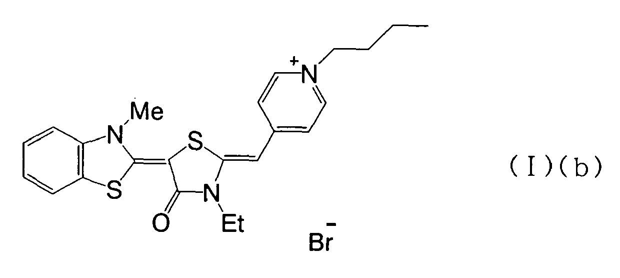

3. A compound shown by formula (I) (c).

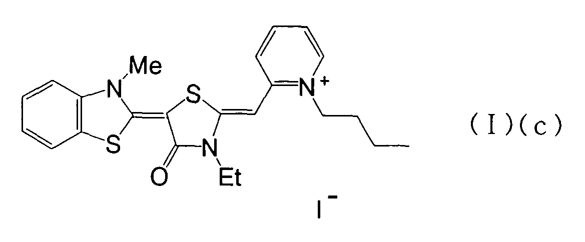

4. A compound shown by formula (I) (d).

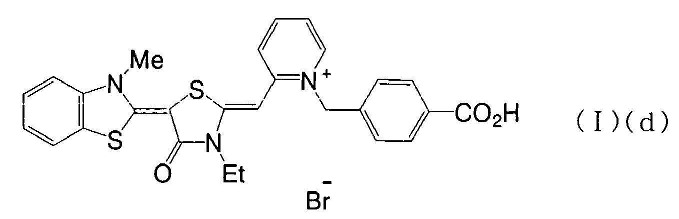

5. An anti-leishmania agent containing a compound shown by general formula (II) as an active ingredient. 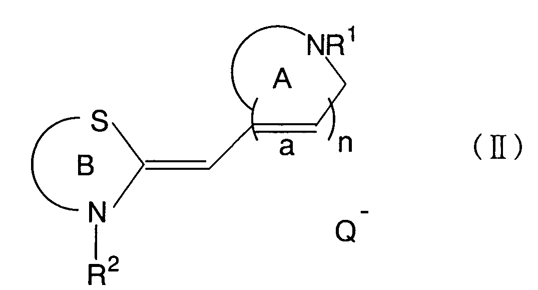 [wherein R¹ represents an alkyl group with 1 to 8 carbon atoms having unsubstituted or substituted group, an aryl group with 6 to 8 carbon atoms having unsubstituted or substituted group, or a heterocyclic group having unsubstituted or substituted group; R² represents a hydrogen atom, an alkyl group with 1 to 8 carbon atoms having unsubstituted or substituted group, an aryl group with 6 to 8 carbon atoms having unsubstituted or substituted group; or a heterocyclic group having unsubstituted or substituted group; n represents an integer of 0, 1 or 2; A and B represent independently an atom group necessary to form a 5- to 8-membered heterocycle having unsubstituted or substituted group; a represents a conjugated system; and Q represents a pharmaceutically acceptable anion]

6. The anti-leishmania agent according to claim 5, wherein the compound shown by general formula (II) is a rhodacyanine dye compound shown by general formula (III). 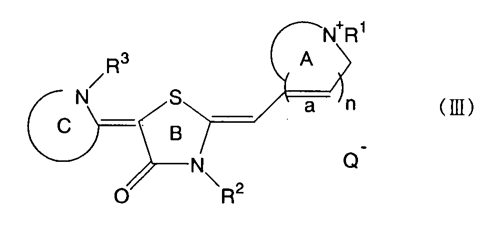 [wherein R¹ represents an alkyl group with 1 to 8 carbon atoms having unsubstituted or substituted group, an aryl group with 6 to 8 carbon atoms having unsubstituted or substituted group, or a heterocyclic group having unsubstituted or substituted group; R² and R³ represent independently a hydrogen atom, an alkyl group with 1 to 8 carbon atoms having unsubstituted or substituted group, an aryl group with 6 to 8 carbon atoms having unsubstituted or substituted group; or a heterocyclic group having unsubstituted or substituted group; n represents an integer of 0, 1 or 2; A and C represent independently an atom group necessary to form a 5- to 8-membered heterocycle having unsubstituted or substituted group; a represents a conjugated system; and Q represents a pharmaceutically acceptable anion]

7. The anti-leishmania agent according to claim 6, wherein the rhodacyanine dye compound shown by general formula (III) is a rhodacyanine dye compound shown by any one of formulae (I) (a) to (d), or formulae (IV) (a) to (m).

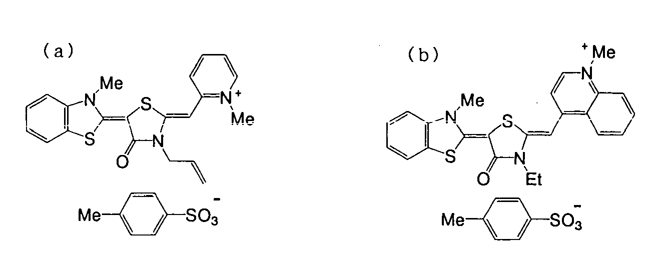

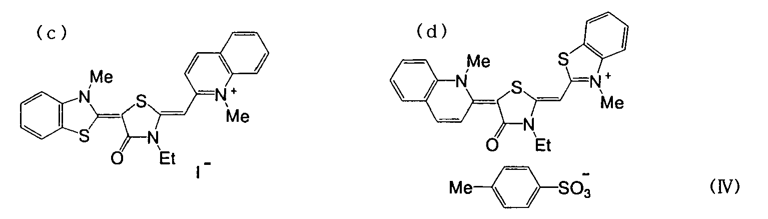

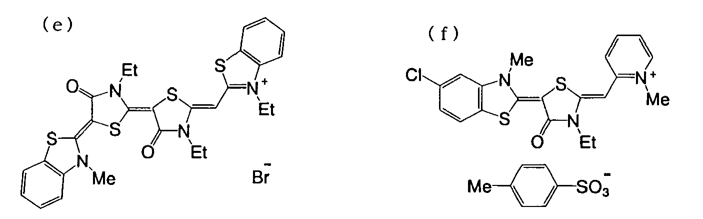

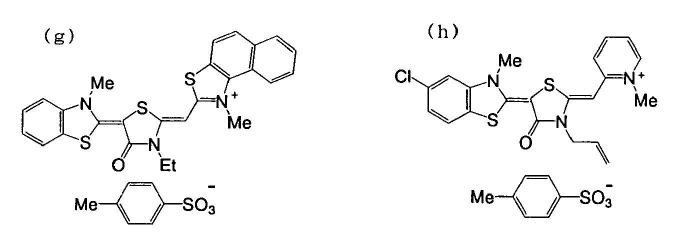

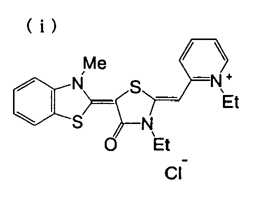

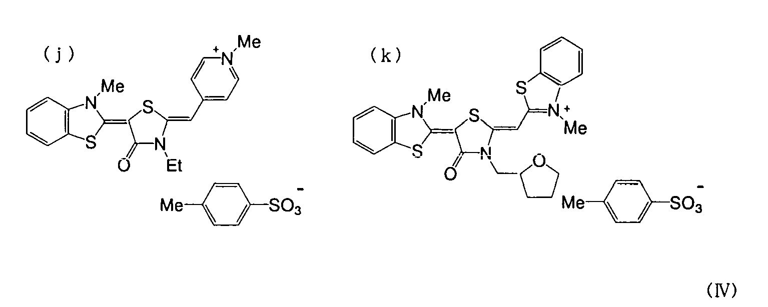

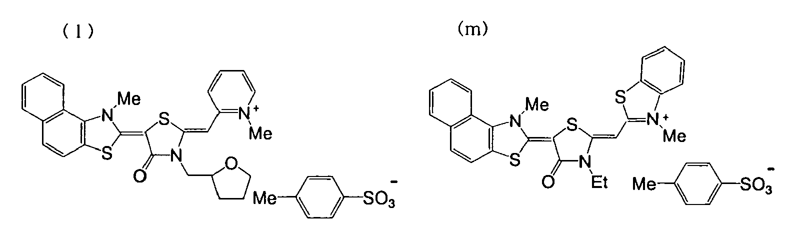

8. The anti-leishmania agent according to claim 5, wherein the compound shown by general formula (II) is a compound shown by formula (V) (a) or (b).
